## Europäisches Patentamt
## European Patent Office
## Office européen des brevets

(19)

(11) Numéro de publication: **0 035 932**
A1

(12)

# DEMANDE DE BREVET EUROPEEN

(21) Numéro de dépôt: **81400319.0**

(22) Date de dépôt: **02.03.81**

(51) Int. Cl.³: **A 61 N 1/42**
**C 12 N 13/00**

(30) Priorité: **03.03.80 FR 8004669**

(43) Date de publication de la demande:
**16.09.81 Bulletin 81/37**

(84) Etats contractants désignés:
**AT BE CH DE GB IT LI LU NL SE**

(71) Demandeur: **FLUX YANG Societé Civile**
**1, rue du Bourg-Neuf**
**F-77160 Provins (Seine et Marne)(FR)**

(72) Inventeur: **Phely, Jean-Pierre**
**1, rue du Bourg-Neuf**
**F-77160 Provins (Seine et Marne)(FR)**

(74) Mandataire: **Rataboul, Michel**
**69, rue de Richelieu**
**F-75002 Paris(FR)**

(54) Dispositif pour l'application d'un champ magnétique.

(57) Le dispositif comprend un aimant permanent (4) associé à un organe de plus grande étendue (2) constituant un diffuseur de champ magnétique associés l'un à l'autre de telle sorte que la face de l'organe (2) devant être dirigée vers l'objet ait une polarité unique sur l'ensemble de sa surface tandis que la face opposée est entièrement de polarité contraire.

Fig.1

EP 0 035 932 A1

## DISPOSITIF POUR L'APPLICATION D'UN CHAMP MAGNETIQUE

La présente invention concerne un dispositif d'emploi
très simple grâce auquel il est possible à toute personne
d'utiliser les effets de champs magnétiques sur le corps
humain, par exemple.

On sait que selon la théorie chinoise traditionnelle,
le corps des êtres vivants, et tout particulièrement le
corps humain, est le siège d'une circulation d'énergie.

Cette énergie se présenterait sous deux formes : INN
et YANG, et circulerait selon des méridiens, lesquels sont
constitués par des lignes virtuelles joignant des points sur
lesquels on peut agir.

L'acupuncture agit généralement en piquant ces points
avec des aiguilles, mais il existe d'autres procédés connus.

Ainsi, selon la technique dite des "Moxas", on agit
également par la chaleur en plaçant à quelques millimètres
au-dessus du point ou de la zone à traiter un cigare de
feuilles d'armoise séchées qui brûlent lentement.

Selon un autre procédé, on agit en exerçant des massages ou des pressions avec l'extrémité des doigts sur des
zones ou sur des points précis.

La très ancienne théorie de l'acupuncture trouve chaque
jour de nouvelles confirmations au fur et à mesure des progrès de la science.

C'est ainsi que l'électro-magnétisme associe les concepts
vibratoires et rotatifs au moyen des moments cinétiques, ou
spins attachés, soit au noyau atomique (spins nucléaire),
soit à l'électron. Ces moments cinétiques résultent de la rotation de la particule sur elle-même et, par suite des charges qu'elle porte, il correspond au spin un moment bipolaire
magnétique. La particule se comporte à la fois comme un gyros- scope et comme un petit aimant. A cette description classique, la mécanique quantique ajoute la quantification spatiale selon laquelle l'axe de rotation ne peut prendre, par rapport au champ apliqué, qu'un nombre limité de positions.

Selon une autre formulation, on pourrait dire que la matière, sous sa forme élémentaire de particules, ne serait que de l'énergie concentrée : un enroulement de rayonnements.

Récemment, on a montré l'effet de champs magnétiques créés par des aimants permanents..

L'expérience montre qu'un effet existe mais, jusqu'à présent, les aimants étaient considérés comme ayant chacun une action globale qui se manifestait sans différentiation du pôle nord et du pôle sud ou de la perpendiculaire à la ligne des pôles.

En outre, aucun effet n'avait été étudié et exploité à l'égard d'objets inanimés, de végétaux ou d'êtres vivants autres que l'Homme.

Les bases scientifiques ont, bien entendu, pu faire l'objet d'études de laboratoire mais, dans la réalité on ne dispose d'aucun moyen pratique pour la mise en oeuvre et l'exploitation industrielle des connaissances scientifiques.

La présente invention apporte une solution à ces divers problèmes et sera bien comprise par la description détaillée ci-après faite en référence au dessin annexé. Bien entendu, la description et le dessin ne sont donnés qu'à titre d'exemple indicatif et non limitatif.

La figure 1 montre l'application de l'invention à un bracelet.

La figure 2 montre un autre mode de réalisation de l'invention appliqué à des aiguilles d'acupuncture.

La figure 3 montre l'application de l'invention à des semelles.

La figure 4 montre une vue schématique d'un mode de réalisation de l'invention appliqué à certains objets.

La figure 5 montre schématiquement l'application de l'invention à un dispositif inventé par la demanderesse mais qui ne fait pas partie de la présente invention.

L'invention concerne un dispositif pour l'application d'un champ magnétique sur un objet, qu'il s'agisse d'un article inanimé, ou de matière organique : soit un être complet tel qu'un Homme, un animal, une plante, soit des fragments tels que des aliments et analogues, caractérisé en ce qu'il comprend au moins un aimant permanent et un organe de plus grande étendue constituant un diffuseur de champ magnétique, associés l'un à l'autre de telle sorte que la face de l'organe devant être dirigée vers l'objet ait une polarité unique sur l'ensemble de sa surface tandis que la face opposée est entièrement de polarité contraire.

Selon un mode de réalisation de l'invention, l'organe est proportionnellement mince par rapport à l'étendue de sa surface et ses deux grandes faces sont chacune d'une polarité unique et toutes deux contraires.

Selon d'autres caractéristiques de l'invention :
- l'organe est constitué par un article devant être au contact d'aliments,
- l'organe est un contenant,
- l'organe est un article tel que support, plaque, grillage, embase, bande et analogue,
- l'organe est de la terre pour des végétaux et au moins un aimant doit être en contact avec cette terre, soit en y étant directement confiné, soit en étant rendu solidaire d'un pot, bac ou analogue, contenant la terre, le pôle Nord du ou des aimants étant dirigé vers les végétaux,
- l'organe est un tore formé par un fil métallique conformé en spirale pour constituer un bracelet, un collier, une ceinture ou analogue,
- l'aimant et l'organe sont intimement associés pour constituer un matériau composite comprenant de la ferrite et un produit de préférence souple tel que de la mousse pour matelas, sièges, revêtements, coussins, semelles et analogues,
- le dispositif est appliqué à la modification d'une activité bactériologique notamment lors de la croissance de végétaux, lors de la conservation, du transport, de la présentation, du conditionnement et de l'emballage de produits organiques tels que des aliments et, notamment, des produits carnés, des produits lactés, des fruits et des légumes, lors de la désinfection de plaies.

Un dispositif conforme à l'invention permet de garantir l'unipolarité du champ auquel on soumet l'objet.

On voit que l'invention assure la bonne orientation des aimants pour que le pôle voulu (soit le pôle nord, soit le pôle sud) soit dirigé vers l'objet et cela en fonction du résultat recherché.

A l'égard de l'Homme on s'est aperçu en effet, que le pôle nord, lorsqu'il est en regard de la peau, a une action sur l'énergie YANG plutôt que sur l'énergie INN tandis que le pôle sud a un effet inverse.

A titre d'exemple, on peut indiquer que les désagréments d'ordre musculaire doivent être traités par le pôle nord (énergie de caractère YANG), tandis que les malaises plus profonds correspondant à des organes internes sont plus sensibles à l'action du pôle sud (énergie de caractère INN).

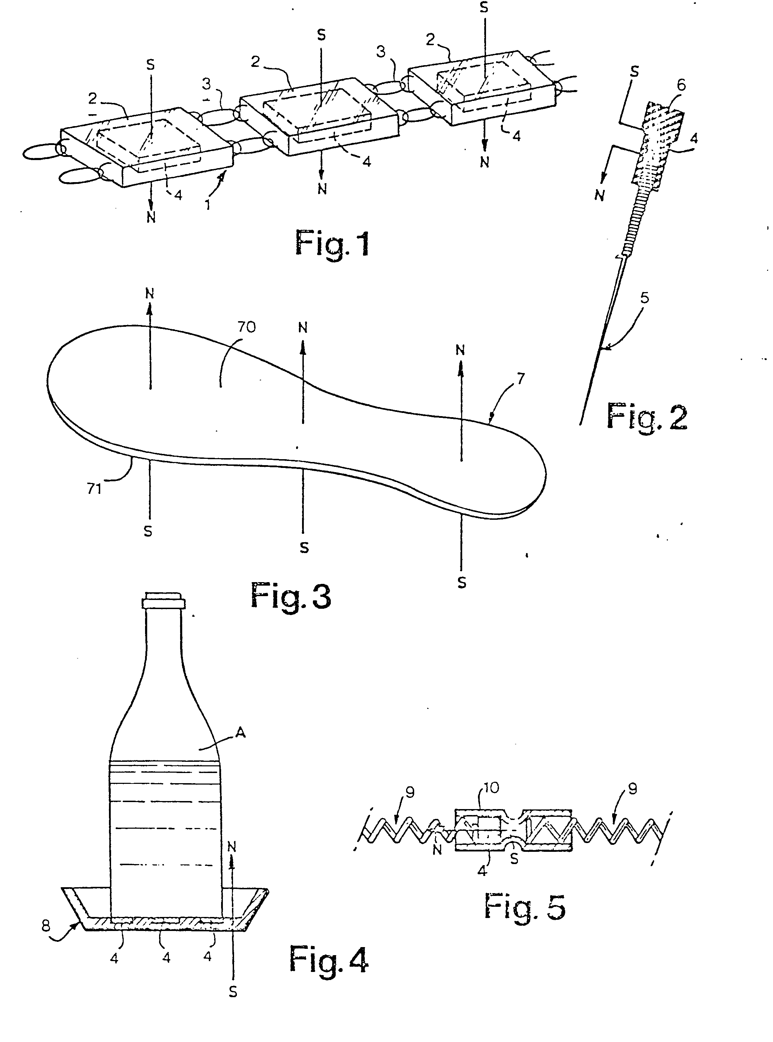

Sur la figure 1 on a représenté un mode de réalisation de l'invention selon lequel un organe est constitué par un bracelet, ou un collier 1 composé d'éléments 2 réunis par une chaînette 3 et contenant, dans leurs masses, des aimants 4.

La matière constitutive des éléments 2 doit avoir des propriétés telles que les effets magnétiques sont convenablement développés, c'est-à-dire diffusés.

Lorsque la chaînette 3 et/ou le système de fermeture du bracelet ou collier 1 permettent sa réversibilité, il faut prévoir un repère extérieur grâce auquel on sait quelle est l'orientation des aimants 4 par rapport aux deux faces de l'organe.

L'aimant 4 peut être réalisé en toute matière voulue mais l'expérience a montré que la matière constitutive a une action importante non seulement sur l'intensité du champ développé mais aussi sur les effets physiologiques. Ces effets sont du genre de ceux procurés par les oligo-éléments.

Un tel effet est particulièrement sensible avec un matériau dans lequel on peut engendrer un champ relativement élevé, ce qui n'est réalisable que s'il possède une haute anisotropie magnétique.

On a pu vérifier que de telle propriétés sont obtenues avec des liaisons inter-métalliques entre des terres rares et du cobalt par exemple.

Les recherches montrent que les liaisons les plus prometteuses sont du type "TRCO R" avec les terres rares Y (Ytrium) La (Lanthane), Ce (Cérium), Pr (Praséodyme), Nd (Néodyme), Sm (Samarium) et leurs mélanges.

Selon les résultats recherchés, il peut être préférable d'utiliser des champs plus faibles, engendrés par exemple, par de petits blocs à base d'oxyde de fer, de baryum ou de strontium orientés convenablement.

Il est possible de constituer l'organe par une aiguille d'acupuncture classique formant diffuseur des effets magnétiques unipolaires, du fait de sa masse bien supérieure à celle de l'aimant lui-même.

Sur la figure 2 on a représenté un mode de réalisation selon lequel une aiguille d'acupuncture courante 5 reçoit un capuchon 6 qui maintient au sommet de l'aiguille 5 un petit aimant 4 de telle manière que ce dernier soit au contact du métal constitutif de l'aiguille et que le capuchon 6 ne gêne pas le manipulateur.

Avec ces dispositions, on combine les avantages de l'acupuncture traditionnelle et les effets magnétiques qui en renforcent l'action.

Lorsque l'on doit placer un dispositif conforme à l'invention sur une zone inclinée du corps d'un homme, d'un animal ou d'une plante, il faut empêcher qu'il glisse.

Dans ce but, on peut prévoir au centre de l'organe diffuseur une petite pointe qui, sans jouer exactement le même rôle qu'une aiguille d'acupuncture, exerce une action physique sur le point à traiter et qui s'oppose à tout déplacement du dispositif, même si cette pointe est à peine enfoncée dans l'épiderme.

Sur la figure 3 on a représenté une semelle 7 destinée à être placée de manière amovible dans une chaussure, pantoufle et analogues, et constituée d'une face supérieure 70, par exemple en textile, et d'une feuille inférieure 71 en mousse d'élastomère contenant de la ferrite aimantée, de telle manière que la polarité de cette feuille corresponde au
nord pour sa face supérieure et au sud pour sa face inférieure.

Ainsi l'usager reçoit les effets magnétiques du pôle
nord et non ceux du pôle sud.

Tous les exemples de réalisation qui ont été décrits
ci-dessus correspondent au traitement direct d'organismes
vivants et tout particulièrement des hommes.

Sur la figure 4 on a représenté un exemple d'application de l'invention à un objet inanimé tel qu'une coupelle
8 dans laquelle on peut placer des aliments ou, comme représenté, de l'eau minérale dans une bouteille A.

Avec ces dispositions, on soumet les aliments ou l'eau
minérale à l'action du flux magnétique des aimants 4 enchassés dans la matière constitutive de la coupelle 8.

Comme dans le cas de la figure 3, on pourrait associer
à la coupelle 8 non plus des aimants indépendants 4 mais une
feuille convenablement découpée et aimantée dans le sens
voulu.

Naturellement, la figure 4 ne représente qu'un simple
exemple d'application de l'invention à un objet inanimé et,
selon les cas d'utilisation pratique, on peut appliquer
l'invention à d'autres objets tels que pots de fleurs, dessous de plat, soucoupes, contenants, etc... Un cas particulièrement intéressant est celui de matelas et coussins grâce
auxquels un usager peut être traité lors de son repos.

En se reportant maintenant à la figure 5, on voit un
mode de réalisation selon lequel l'invention est appliquée
à un dispositif inventé par la demanderesse mais qui ne fait
pas partie de la présente invention.

Ce dispositif comprend un tore 9 dont les extrémités
sont maintenues par une bague 10.

Ici on place à l'intérieur de la bague 10 un aimant 4
qui est au contact d'une extrémité du tore par le pôle nord.

Dans certains cas, on doit développer un champ magnétique beaucoup moins intense que celui qui résulte des aimants
que l'on a décrit ci-dessus.

On peut alors utiliser de la poudre de ferrite agglomérée par un liant synthétique souple dès lors que l'une des faces ne présente face à l'objet qu'un pôle unique. Cette disposition est contraire à celle, habituelle, de ce genre de matériau qui présente une succession de pôles différents disposés le long d'une bande.

On peut appliquer l'invention à la croissance de végétaux ou autres organismes.

Ainsi, selon l'invention, un aimant au moins est associé à un diffuseur qui est appliqué à la culture de moisissures ou au traitement de micro-organismes, soit pour en favoriser la croissance, soit pour en limiter l'activité, notamment en vue de la conservation d'aliments ou en vue de la désinfection de plaies.

On voit que l'invention permet d'établir sélectivement la polarité des aimants selon le type d'action recherché.

Ainsi on utilise le pôle nord dont l'action est plus favorable au développement de l'énergie sous ses formes dites de "circulation INN" et de "circulation YANG" selon les lois de l'acupuncture. D'une façon pratique cette action se traduit notamment par une aide importante de l'élimination des contractions musculaires.

Il est donc important que l'usager soit assuré de ne pas se tromper quant au sens du flux magnétique favorable, afin que seul le pôle voulu soit en contact avec l'objet à traiter et, en tout premier lieu avec le corps humain.

Afin que l'usager ne se trompe pas et place les pôles dans l'orientation voulue, on prévoit que l'une au moins des deux faces de l'aimant est munie d'un repère indiquant sa polarité.

Ainsi, une face peut être peinte d'une certaine couleur pour indiquer le pôle nord, l'autre étant ipso facto repérée comme étant le pôle sud. On pourrait aussi marquer les faces, par exemple : la lettre "N" pour le pôle nord et/ou la lettre "S" pour le pôle sud.

L'usager peut de la sorte correctement orienter l'aimant lui-même soit pour le retourner lorsqu'il est utilisé avec un support de tout type connu, soit pour le placer selon l'orientation voulue lorsqu'il est utilisé indépendemment et maintenu en place par un ruban adhésif par exemple.

L'invention n'est pas limitée aux seuls modes de réalisation décrits et représentés, mais en embrasse au contraire toutes les variantes.

REVENDICATIONS

1 - Dispositif pour l'application d'un champ magnétique sur un objet, qu'il s'agisse d'un article inanimé, ou de matière organique : soit une être complet tel qu'un Homme, un animal, une plante, soit des fragments tels que des aliments et analogues, caractérisé en ce qu'il comprend au moins un aimant permanent (4) et un organe de plus grande étendue (2-5-7-8) constituant un diffuseur de champ magnétique associés l'un à l'autre de telle sorte que la face de l'organe devant être dirigée vers l'objet ait une polarité unique sur l'ensemble de sa surface tandis que la face opposée est entièrement de polarité contraire.

2 - Dispositif selon la revendication 1, caractérisé en ce que l'organe (7) est proportionnellement mince par rapport à l'étendue de sa surface et ses deux grandes faces (70 et 71) sont chacune d'une polarité unique et toutes deux contraires.

3 - Dispositif selon la revendication 1, caractérisé en ce que l'organe est constitué par un article (8) devant être en contact d'aliments.

4 - Dispositif selon la revendication 1, caractérisé en ce que l'organe est un contenant.

5 - Dispositif selon la revendication 1, caractérisé en ce que l'organe est un article tel que support, plaque, grillage, embase, bande et analogue (8).

6 - Dispositif selon la revendication 1, caractérisé en ce que l'organe est de la terre pour des végétaux et qu'au moins un aimant (4) doit être en contact avec cette terre soit en y étant directement confiné, soit en étant rendu solidaire d'un pot, bac ou analogue contenant la terre, le pôle nord du ou des aimants étant dirigé vers les végétaux.

7 - Dispositif selon la revendication 1, caractérisé en ce que l'organe est un tore (9) formé par un fil métallique conformé en spirale pour constituer un bracelet, un collier, une ceinture ou analogue.

8 - Dispositif selon la revendication 1, caractérisé en ce que l'aimant et l'organe (7) sont intimement associés pour constituer un matériau composite comprenant de la ferrite et un produit de préférence souple tel que de la mousse pour matelas, sièges, revêtements, coussins, semelles et analogues.

9 - Dispositif selon la revendication 1, caractérisé en ce qu'il est appliqué à la modification d'une activité bactériologique notamment lors de la croissance de végétaux, lors de la conservation, du transport, de la présentation, du conditionnement et de l'emballage de produits organiques tels que des aliments et, notamment, des produits carnés, des produits lactés, des fruits et des légumes, lors de la désinfection de plaies.

PL.1/1

Fig. 1

Fig. 2

Fig. 3

Fig. 4

Fig. 5

**0035932**

Office européen des brevets

**RAPPORT DE RECHERCHE EUROPEENNE**

Numéro de la demande

EP 81 40 0319

| Catégorie | Citation du document avec indication, en cas de besoin, des parties pertinentes | Revendication concernée |
|---|---|---|
| **DOCUMENTS CONSIDERES COMME PERTINENTS** | | |
| X | DE - A - 2 308 927 (MANSK)<br>* Page 3, lignes 9-16; page 4, lignes 11-16; figures * | 1,2,5,7 |
| | -- | |
| X | FR - A - 1 450 093 (ROSSET)<br>* Page 1, colonne de droite, ligne 17 à page 2, colonne de gauche, ligne 2; figures 1,2 * | 1,2,5,8 |
| | -- | |
| | FR - A - 1 306 484 (WELEDA)<br>* Page 1, colonne de droite, ligne 27 à page 2, colonne de gauche, ligne 21; figure 1 * | 1,5 |
| | -- | |
| | FR - A - 2 423 233 (T.D.K. ELECTRONICS)<br>*´ Page 5, lignes 17-33; figures 4-6 * | 1,2,5,7 |
| | -- | |
| | FR - A - 781 083 (BOVIS)<br>* Page 2, lignes 45-85; figures 4-8 * | 1-6,9 |
| | -- | |
| | DE - A - 2 733 982 (WIBO-WERK)<br>* Page 7, ligne 5 à page 8, ligne 3; figures 3,4 * | 1,2,5,7,8 |
| | -- | |
| | US - A - 4 162 672 (FUJIMOTO)<br>* Colonne 2, lignes 27-52; colonne 3, lignes 37-58; ./. | 1,2,5 |

**CLASSEMENT DE LA DEMANDE (Int. Cl.³)**

A 61 N    1/42
C 12 N   13/00

**DOMAINES TECHNIQUES RECHERCHES (Int. Cl.³)**

A 61 N    1/42

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

Le présent rapport de recherche a été établi pour toutes les revendications

| Lieu de la recherche | Date d'achèvement de la recherche | Examinateur |
|---|---|---|
| La Haye | 03-06-1981 | BIJN |

OEB Form 1503.1   06.78

**Office européen des brevets**

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée |
|---|---|---|
| | figures 1-6 * | |
| | -- | |
| PE | WO - A - 81/00357 (URAGAMI) <br> * Abrégé * | 1,5,8 |
| | ---- | |

**CLASSEMENT DE LA DEMANDE (Int. Cl. 3)**

**DOMAINES TECHNIQUES RECHERCHES (Int. Cl. 3)**

OEB Form 1503.2  05.78